# EUROPEAN PATENT SPECIFICATION

(11) **EP 1 827 500 B1**
(45) Date of publication and mention of the grant of the patent: **06.05.2009**
(21) Application number: 05803151.9
(22) Date of filing: 26.10.2005
(51) Int. Cl.: A61K 47/48, A61K 31/704, A61K 31/4995, A61K 45/06, A61P 35/00

(54) **PEGYLATED LIPOSOMAL DOXORUBICIN IN COMBINATION WITH ECTEINESCIDIN 743**
PEGYLIERTES LIPOSOMALES DOXORUBICIN IN KOMBINATION MIT ECTEINESCIDIN 743
DOXORUBICINE LIPOSOMIALE PEGYLEE EN COMBINAISON AVEC L' ECTEINESCIDINE 743

(30) Priority: 26.10.2004 US 622163 P
(43) Date of publication of application: 05.09.2007
(73) Proprietor: Pharma Mar S.A., Sociedad Unipersonal, 28770 Madrid (ES); Ortho Biotech Products L.P., Raritan NJ 08869 (US)
(72) Inventor: GILLES, Erard, Bridgewater, New Jersey 08807 (US); STERNAS, Lars-Axel, Verona, New Jersey 07044 (US); TRIFAN, Ovid, Wallingford, Connecticut 06492 (US); VAN DE VELDE, Helgi, B-2340 Beerse (BE); TEITELBAUM, April, Doylestown, Pennsylvania 18901 (US)
(74) Representative: Fleck, Barbara
(86) International application number: PCT/GB2005/050189
(87) International publication number: WO 2006/046080

(56) References cited:
- WO-A-02/36135
- WO-A-20/05049029
- ANONYMOUS: "Doxil (doxorubicin HCl liposome injection) product information" INTERNET ARTICLE, [Online] 10 October 2004 (2004-10-10), pages 1-16, XP002389462 Retrieved from the Internet: URL:http://web.archive.org/web/20041009180 801/www.doxil.com/05_shared_pages/01_presc ribing_info.html> [retrieved on 2006-07-10]
- LAVERDIERE CAROLINE ET AL: "Phase II study of ecteinascidin 743 in heavily pretreated patients with recurrent osteosarcoma" CANCER, AMERICAN CANCER SOCIETY, PHILADELPHIA, PA, US, vol. 98, no. 4, 15 August 2003 (2003-08-15), pages 832-840, XP002314512 ISSN: 0008-543X
- TAKAHASHI N ET AL: "Sequence-dependent enhancement of cytotoxicity produced by ecteinascidin 743 (ET-743) with doxorubicin or paclitaxel in soft tissue sarcoma cells" CLINICAL CANCER RESEARCH, THE AMERICAN ASSOCIATION FOR CANCER RESEARCH, US, vol. 7, no. 10, October 2001 (2001-10), pages 3251-3257, XP002314514 ISSN: 1078-0432
- SESSA C. ET AL: "Trabectedin for women with ovarian carcinoma after treatment with platinum and taxane fails" JOURNAL OF CLINICAL ONCOLOGY, vol. 23, no. 9, 20 March 2005 (2005-03-20), pages 1867-1874, XP002389463

## Description

The present invention relates to the treatment of cancers and, in particular, an effective treatment of human cancers using Ecteinascidin 743 (ET-743) in combination with another drug.

Ecteinascidin 743 (ET-743) is an anticancer agent derived from a marine source.

### BACKGROUND OF THE INVENTION

Ecteinascidin 743 (ET-743) is a tetrahydroisoquinoline alkaloid isolated from the marine tunicate *Ecteinascidia turbinata* with the following structure:

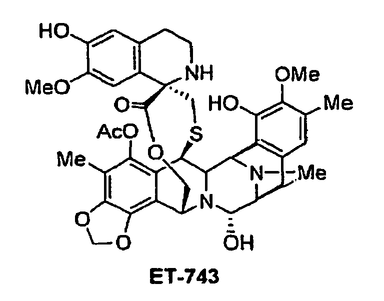

ET-743, its chemistry, mechanism of action and preclinical and clinical development can be found in Kesteren, Ch. Van et al., 2003, Anti-Cancer Drugs, 14 (7), pages 487-502: "Yondelis (trabectedin, ET-743): the development of an anticancer agent of marine origin", and references therein.

ET-743 possesses potent antineoplastic activity against a variety of human tumour xenografts grown in athymic mice, including melanoma and ovarian and breast carcinoma.

In clinical phase I studies of ET-743, promising responses were observed in patients with sarcoma and breast and ovarian carcinoma. Therefore this new drug is currently under intense investigation in several phase II clinical trials in cancer patients with a variety of neoplastic diseases.

ET-743 has myelotoxic and hepatotoxic side effects. Patients who received ET-743 by prolonged infusion over 24-72 hr experienced myelosuppression and, frequently, acute, albeit reversible, elevation of transaminases and subclinical cholangitis characterized by increases in alkaline phosphatase (ALP) and/or bilirubin, see for example Ryan D.P. et al., 2001 Clin Cancer Res 7, 231: "Phase I and pharmacokinetic study of ecteinascidin 743 administered as a 72-hour continuous intravenous infusion in patients with solid malignancies"; Puchalski T.A. et al., 2002, Cancer Chemother Pharmacol 50: 309: "Pharmacokinetics of ecteinascidin 743 administered as a 24-h continuous intravenous infusion to adult patients with soft tissue sarcomas: associations with clinical characteristics, pathophysiological variables and toxicity".

Preclinical acute toxicity studies conducted in mice, rats, dogs and monkeys consistently demonstrated liver toxicity as an important side effect of ET-743, as evidenced by an increase in plasma levels of liver-specific enzymes and pathological manifestations of cholangitis. Recently the nature and extent of the hepatobiliary changes exerted by ET-743 in the female rat, the species which is most susceptible towards the hepatotoxic potential of ET-743, has been characterized by histopathology, electron microscopy, immunohistochemistry, plasma biochemistry and DNA microarray analysis, see Donald S. et al., 2002, Cancer Research, 62: 4256 "Hepatobiliary damage and changes in hepatic gene expression caused by the antitumor drug ecteinascidin 743 (ET-743) in the female raf".

Furthermore, pretreatment with high-dose dexamethasone has been shown to abrogate ET-743-mediated hepatotoxicity in this animal model without impeding its antitumor activity, see Donald S. et al., 2003, Cancer Research, 63: 5903-5908: "Complete protection by high-dose dexamethasone against the hepatotoxicity of the novel antitumor drug ecteinascidin-743 (ET-743) in the rat" and WO 02 36135. Protection by dexamethasone pretreatment was accompanied by a dramatic reduction in hepatic levels of ET-743, tentatively implicating elevated hepatic clearance of ET-743, perhaps *via* induced metabolic enzymes, as the mechanism by which dexamethasone exerts its beneficial effect, ie via an increase in the rate of metabolic detoxification of ET-743.

Doxorubicin is a cytotoxic anthracycline antibiotic isolated from *Streptomyces peucetius* var. *caesius.* Doxorubicin is known to cause primarily myelotoxicity when administered alone.

The reader is referred to WO 02 36135, published 10 May 2002 ,for compositions and uses of ET-743 with other drugs for treating cancer. *In vitro* assays indicated more than additive effects for the combination of ET-743 with several other drugs. In particular, synergistic effects were shown *in vitro* against human sarcoma tumours. Composition and uses of ET-743 with the anthracycline Doxorubicin were investigated. In this study there was no detailed consideration of the toxicity of combinations.

Further guidance on the dosage, schedules and administration of ET-743 alone or in combination is given in WO 00 69441, WO 02 36135, WO 03 039571 and PCT/GB2004/002319

There is still a need to provide further therapies that allow an effective treatment of mammals, in particular humans, with ET-743 while reducing or eliminating its toxic side effects and minimizing further adverse effects.

### DRA WING OF THE INVENTION

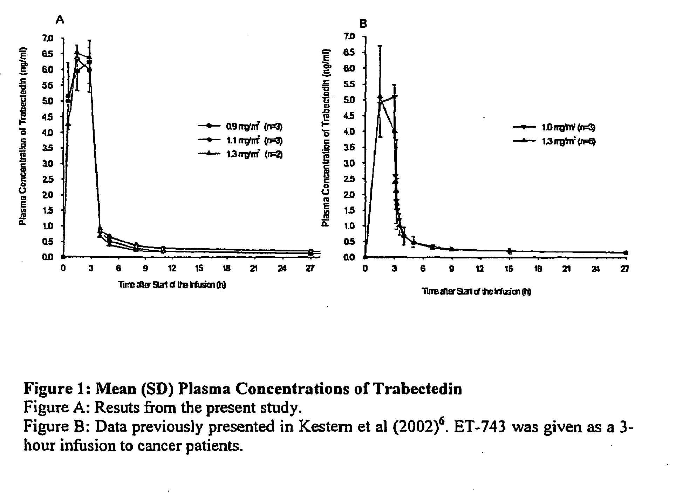

Figure 1 shows the Mean Plasma Concentration of ET-743 (also referred to as Trabectedin throughout the Examples) as a function of time after the start of the infusion, where Figure 1A relates to results obtained from the present study and Figure 1B relates to results presented in Van Kestern et al. ("Clinical Pharmacology of the novel marine-derived anticancer agent Ecteinascidin 743 administered as a 1- and 3-hour infusion in a phase I study; Anticancer Drugs; 13(4); 381-393; 2002").

### SUMMARY OF THE INVENTION

This invention relates to combination products, combination drug treatments and treating patients afflicted with cancer, having fewer and less severe unwanted toxic effects.

In accordance with one aspect, the invention provides a treatment of the human body for cancer comprising administering an effective therapeutic amount of a Pegylated Liposomal form of the anthracycline Doxorubicin ("PLD"), in combination with an effective therapeutic amount of ET-743. Preferably the mammal is a human.

### EMBODIMENTS OF THE INVENTION

Surprisingly, it has been found that the combination of ET743 and PLD can lead to increased anti-tumour efficacy while at the same time having reduced myelotoxicity and reduced cardiotoxicity. Furthermore, the combination of ET-743 with PLD is synergistic.

The PLD is Doxorubicin hydrochloride (HCl) in Pegylated Liposomal form. The encapsulation in liposomes makes it suitable for intravenous administration. Liposomes are microscopic vesicles composed of a phospholipid bilayer that are capable of encapsulating active drugs. "Pegylation" is when liposomes are formulated with surface-bound methoxypolyethylene glycol (MPEG). Liposomal encapsulation may substantially affect a drug's functional properties relative to those of the unencapsulated drug. In addition, different liposomal drug products may vary from one another in the chemical composition and physical form of the liposomes. Such differences may substantially affect the functional properties of liposomal drug products. Doxil™ is an example of a commercially available form of Pegylated Liposomal Doxorubicin.

A combination of PLD and ET-743 is effective with reduced myelotoxicity and reduced cardiotoxicity in comparison with the toxicities observed using a combination of Doxorubicin and ET-743.

The increased anti-tumour efficacy is in comparison to treatments using ET743 alone. It has been found that the combination of PLD and ET-743 is tolerated to an extent in which both drugs may be administrated at full, or near full, therapeutic doses for prolonged periods of time.

In one aspect, the present invention is directed to a composition for the treatment of the human body for cancer, comprising ET-743 and PLD, which is effective with reduced toxicity in comparison with the toxicity observed using a combination of Doxorubicin and ET-743. In particular, the ET-743 and PLD combination shows reduced myelotoxicity and reduced cardiotoxicity.

In another aspect, the present invention is directed to a medical kit for administering ET-743 in combination with PLD, comprising a supply of ET-743 in dosage units for at least one cycle, wherein the dosage unit contains the appropriate amount of ET-743 for the treatments defined and a pharmaceutically acceptable carrier, and printed instructions for administering ET-743 according to a dosing schedule.

In another aspect, the invention is directed to the use of ET-743 in the preparation of a medicament for an effective treatment of the human body for cancer by combination therapy employing ET-743 with PLD.

The term "combination" as used throughout the specification, is meant to encompass the administration of the therapeutic agents in the same or separate pharmaceutical formulations, and at the same time or at different times.

In a further aspect, the invention is directed to the use of PLD in the preparation of a medicament for an effective treatment of the human body for cancer by combination therapy employing PLD with ET-743. The treatment is effective with reduced myelotoxicity and cardiotoxicity and is also notable for the absence of both mucositis and alopecia.

In a further aspect, the present invention is directed to increasing anti-tumour efficacy of ET-743 in a treatment of the human body for cancer comprising administering an effective therapeutic amount of ET-743 in combination with an effective therapeutic amount of PLD.

The invention also provides a treatment of the human body for cancer comprising administering an effective therapeutic amount of PLD in combination with an effective therapeutic amount of ET-743. Preferably the mammal is a human.

The term "ET-743" is intended here to cover any pharmaceutically acceptable salt, ester, solvate, hydrate which, upon administration to the recipient is capable of providing (directly or indirectly) the compound as described herein. However, it will be appreciated that non-pharmaceutically acceptable salts also fall within the scope of the invention since these may be useful in the preparation of pharmaceutically acceptable salts. The preparation of salts can be carried out by methods known in the art.

ET-743 is supplied and stored as a sterile lyophilized product, consisting of ET-743 and excipient in a formulation adequate for therapeutic use, in particular a formulation containing mannitol and a phosphate salt buffered to an adequate pH.

Administration of the compositions is suitably by intravenous injection. Administration can be carried out continuously or periodically within the maximum tolerated dose (MTD). Throughout the specification, MTD is intended to relate to the highest dose at which less than one third of the subjects in a dose-level cohort experienced dose limiting toxicity (DLT).

ET-743 and PLD may be provided as separate medicaments for administration at the same time or at different times. Preferably, ET-743 and PLD are provided as separate medicaments for administration at different times. When administered separately and at different times, either ET-743 or PLD may be administered first; however, it is preferable to administer PLD followed by ET-743.

Typical infusion times are up to 72 hours, more preferably 1-24 hours, with 1-6 hours most preferred. When PLD and ET-743 are provided as separate medicaments for administration at different times, the infusion times for each may differ.

Infusion times for PLD are generally up to 6 hours, more preferably 1-3 hours, with 1-2 hours most preferred. Infusion times for ET-743 are generally up to 24 hours, more preferably about 1, about 3 or about 24 hours. Short infusion times which allow treatment to be carried out without an overnight stay in hospital are especially desirable.

It will be appreciated that the correct dosage of the compositions of this aspect of the invention will vary according to the particular formulation, the mode of application, and the particular *situs,* host and tumour being treated. Other factors like age, body weight, sex, diet, time of administration, rate of excretion, condition of the host, drug combinations, reaction sensitivities and severity of the disease shall be taken into account. All dosages are expressed in milligrams (mg) per metre square (m²) of body surface area. Since in this method of the invention PLD and ET-743 are used in combination, the dosage of each is adjusted to provide the optimum clinical response.

In the present invention, dosages of PLD of up to 50 mg/m² are used, more preferably 25-45 mg/m², with 30-40 mg/m² most preferred, with about 30 mg/m² even most preferred. Dosages of ET-743 of up to 1.3 mg/m² are used, more preferably 0.4-1.2 mg/m², with about 1.1 mg/m² most preferred.

According to a preferred embodiment of this aspect of the invention, 25-45 mg/m² of PLD are administered intravenously followed by up to 1.3 mg ET-743, also administered intravenously. More preferably, about 30 mg/m² of PLD are administered followed by about 1.1 mg/m² ET-743. The PLD is preferably administered over an infusion time of up to 6 hours, more preferably 1-2 hours, most preferably 1 hour. The ET-743 is preferably administered over an infusion time of about 1, about 3 or about 24 hours.

The administration of the combination is performed in cycles in the application method of the invention. Intravenous infusions of PLD and ET-743 are given to the patients typically every 3 weeks, allowing for a resting phase in each cycle in which the patients recover. The preferred duration of each cycle is typically of 3 to 4 weeks; multiple cycles can be given as needed. Dose delays and/or dose reductions and schedule adjustments are performed as needed depending on individual patient tolerance of treatments.

According to a particularly preferred embodiment, every 3 weeks, about 30 mg/m² of PLD are administered to a patient over an infusion time of about 1 hour followed by administration of about 1.1 mg/m² of ET-743 over an infusion time of about 3 hours

By using a dosing regimen in accordance with that used in these preferred embodiments, it has been found that the combination is well tolerated when both drugs are administrated at full, or near full, therapeutic doses for prolonged periods of time.

The full dose of ET-743 is known to be 1.3 mg/m² when administered as a single agent over 3 hours. The full dose of PLD as it is currently used in clinical practice is 10 mg/m² per week when administered as a single agent.

The following figures indicate that the use of PLD in combination with ET-743, allows an escalated dose of ET-743 to be tolerated, in comparison to when Doxorubicin is used. A phase I dose escalation study of Doxorubicin (60 mg/m²) in combination with ET-743, administered over 3 hours, could only support a ET-743 dose of 0.7 mg/m². In a Phase I dose escalation study of 30 mg/m² PLD in combination with ET-743 administered over 3 hours, ET-743 could be escalated to 1.1 mg/m².

Accordingly, in another aspect, the present invention is directed to maximising the tolerated dose of ET-743 in a treatment of the human body for cancer comprising administering an effective therapeutic amount of ET-743 in combination with a Pegylated Liposomal form of Doxorubicin.

In summary, the combination of ET-743 and PLD allows for an effective cancer therapy in humans, with reduced myelotoxicity and cardiotoxicity. In phase I trials using ET-743 together with PLD, measurable responses demonstrated evidence of clinical benefit to patients with soft tissue sarcoma, and ovarian and head and neck cancer.

The markedly reduced cardiotoxicity exhibited means that the combinations for use in this aspect of the invention can be administered on a longterm basis. Furthermore, the combinations are notable for the absence of both mucositis and alopecia.

Example 1 shows the results of a study to evaluate the MTD of ET-743 in combination with 30 mg/m² of PLD, together with results of phase I trials. The MTD of ET743 in combination with 30 mg/m² of PLD was established as 1.1 mg/m² in the course of treatments.

In summary, this invention therefore provides a treatment, the use of the compounds in the preparation of a composition for treatment of cancer and related embodiments. The present invention also extends to the compositions of the invention for use in a treatment.

The present invention also relates to pharmaceutical preparations including a pharmaceutically acceptable carrier, which contain as active ingredient a compound or compounds of the invention, as well as the processes for their preparation.

The following example further illustrates the invention.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

### EXAMPLES OF THE INVENTION

Throughout the Example Ecteinascidin 743 (ET-743) is also referred to as Trabectedin.

### Example 1

A phase I trial combining PLD and trabectedin was performed. The objective of this study was to determine the maximum tolerated dose (MTD) of trabectidin in combination with PLD 30 mg/m² administered every 21 days. Additional objectives were to evaluate the safety profile of this combination of drugs and to evaluate the pharmacokinetics of trabectidin and PDL when given in combination. The maximum tolerated dose (MTD) relates to the highest dose at which less than one third of the subjects in a dose-level cohort experienced dose-limiting toxicity (DLT).

We designed a dose finding trial with a fixed PLD dose of 30 mg/m² administered intravenously over one hour, followed immediately by one of six trabectedin doses (0.4, 0.6, 0.75, 0.9, 1.1, and 1.3 mg/m²) administered intravenously over 3 hours. This treatment was repeated every 21 days.

Entry criteria included normal liver function tests, limited prior doxorubicin exposure (dose less than 250 mg/m²), normal cardiac function and Eastern Cooperative Oncology Group (ECOG) Performance Status (PS) score of 0 or 1.

Thirty patients, 14 with sarcomas, 2 with ovarian cancer, 2 with pancreatic cancer, 2 with head and neck carcinoma, 1 with bladder cancer, 1 with breast cancer, 1 with gastric cancer, 1 with NSCLC, 1 with SCLC and 5 more with other types of cancers have been treated (Table 1).

**Table 1: Baseline Disease Characteristics**

| **Time to 1st Dose Since Diagnosis, Mo** | |
|---|---|
| N | 30 |
| Mean (SD) | 37.4 (43.8) |
| Median | 25.5 |
| Range | 1.2 - 216.0 |

| **Time to 1st Dose Since Last Chemo, Mo** | |
|---|---|
| N | 22 |
| Mean (SD) | 3.54(3-0) |
| Median | 2.6 |
| Range | 0.9 - 12.8 |

| **Histology** | |
|---|---|
| N | 30 |
| Bladder | 1 |
| Breast | 1 |
| Head and | 2 |
| Neck | |
| NSCLC | 1 |
| Ovary | 2 |
| Pancreatic | 2 |
| Sarcoma | 14 |
| Gastric | 1 |
| SCLC | 1 |
| Other | 5 |

Nine patients of 30 had a Performance Status (PS) of 0 (Fully active, able to carry on all pre-disease performance without restriction). Table 2 shows demographic data of the patients treated.

**Table 2 Demographic Data**

| | Total |
|---|---|
| **Sex, n (%)** | |
| N | 30 |
| Female | 17 (57) |
| Male | 13 (43) |
| **Race, n (%)** | |
| N | 30 |
| Black | 1 (3) |
| White | 29 (97) |
| **Age in Years** | |
| N | 30 |
| Category, n (%) | |
| 18 - 60 | 25 (83) |
| >60 | 5 (17) |
| Mean (SD) | 51.5(13.8) |
| Median | 53.0 |
| Range | 20 - 78 |
| **Baseline ECOG Score, n (%)** | |
| N | 30 |
| 0 | 9(30) |
| 1 | 21 (70) |

Patients were heavily pretreated: 23/30 had received 1-5 prior chemotherapies (median 3), 15/30 prior radiotherapy, and 27/30 prior surgical resection (Table 3).

**Table 3: Previous Therapy**

| | N=30 |
|---|---|
| **Previous Systemic Therapy, n (%)** | |
| No | 7 (23) |
| Yes | 23 (77) |
| **Previous Surgery, n (%)** | |
| No | 3(10) |
| Yes | 27 (90) |
| **Previous Radiotherapy, n (%).** | |
| No | 15(50) |
| Yes | 15(50) |

Tables 4a and 4b show the number (N) of patients exposed in each ET-743 dose, the treatment duration and the dose intensity.

**Table 4a: Exposure to Treatment: Treatment Duration and Dose Intensity**

| | ET743 0.4 mg/m² (N=3) | ET743 0.6 mg/m² (N=3) | ET743 0.75 mg/m² (N=3) |
|---|---|---|---|
| **Total Treatment Duration, Weeks** | | | |
| Mean (SD) | 39.4 (37.4) | 31.5 (37.1) | 24.2 (31.7) |
| Median | 24.0 | 14.4 | 6.0 |
| Range | 12.1 - 82.0 | 6.0 - 74.0 | 5.9 - 60.9 |
| **Total Number of Cycles** | | | |
| Mean (SD) | 13.0 (12.3) | 10.0 (12.2) | 7.7 (9.8) |
| Median | 8.0 | 4.0 | 2.0 |
| Range | 4 - 27 | 2 - 24 | 2 - 19 |
| **Overall Relative Dose Intensity ET-743** | | | |
| Mean (SD) | 1.1 (0.2) | 0.9 (0.1) | 1.0 (0.0) |
| Median | 1.0 | 1.0 | 1.0 |
| Range | 1.0 -1.3 | 0.8 - 1.0 | 0.9 - 1.0 |
| **Overall Relative Dose Intensity PLD** | | | |
| Mean (SD) | 0.9 (0.1) | 0.9 (0.1) | 0.9 (0.2) |
| Median | 1.0 | 1.0 | 1.0 |
| Range | 0.8 -1.0 | 0.8 - 1.0 | 0.7-1.0 |

**Table 4b: Exposure to Treatment: Treatment Duration and Dose Intensity**

| | ET743 0.9 mg/m² (N=3) | ET743 1.1 mg/m² (N=12) | ET743 1.3 mg/m² (N=6) | Total (N=30) |
|---|---|---|---|---|
| **Total Treatment Duration, Weeks** | | | | |
| Mean (SD) | 6.0 (0.0) | 14.6 (11.1) | 25.7 (11.3) | 21.1 (20.5) |
| Median | 6.0 | 11.1 | 25.5 | 13.0 |
| Range | 6.0-6.0 | 3.0-42.0 | 7.1-39.9 | 3.0-82.0 |
| **Total Number of Cycles** | | | | |
| Mean (SD) | 2.0 (0.0) | 4.8 (3.8) | 8.2 (3.8) | 6.8 (6.7) |
| Median | 2.0 | 3.5 | 8.0 | 4.0 |
| Range | 2 - 2 | 1 - 14 | 2-13 | 1-27 |
| **Overall Relative Dose Intensity ET-743** | | | | |
| Mean (SD) | 1.1 (0.0) | 0.9(0.1) | 0.8(0.1) | 0.9(0.1) |
| Median | 1.0 | 0.9 | 0.7 | 1.0 |
| Range | 0.9 - 1.0 - | 0.8 - 1.0 | 0.7 -1.0 | 0.7 -1.3 |
| **Overall Relative Dose Intensity PLD** | | | | |
| Mean (SD) | 1.0 (0.0) | 0.9(0.1) | 0.8(0.1) | 0.9(0.1) |
| Median | 1.0 | 0.9 | 0.7 | 0.9 |
| Range | 0.9-1.0 | 0.7-1.0 | 0.7-0.9 | 0.7-1.0 |

Worst on-treatment drug related grade 3 and 4 toxicities have been minimal, limited to neutropenia and transaminases elevation. Tables 5a and 5b show the frequently reported drug-related Grade 3/4 adverse events in least 5% of subjects. The adverse events reported at any time from first treatment dose up to 30 days after the last treatment dose are included. In order to define the toxicity grade, NCI common criteria are used.

**Table 5a Drug Related Adverse Events**

| | ET743 0.4 mg/m² (N=3) | ET743 0.6 mg/m² (N=3) | ET743 0.75 mg/m² (N=3) |
|---|---|---|---|
| **Total no. subjects with** | | | |
| **Grade 3/4 drug-** | | | |
| **related AE** | 0 | 1 | 0 |
| **Liver and Biliary** | 0 | 0 | 0 |
| **System** | | | |
| SGPT Increased | 0 | 0 | 0 |
| SGOT Increased | 0 | 0 | 0 |
| **Other** | 0 | 1 | 0 |
| Palmar-Plantar | | | |
| Erythrodysesthesia | 0 | 0 | 0 |
| Allergic Reaction | 0 | 0 | 0 |
| Nausea | 0 | 1 | 0 |

**Table 5b Drug Related Adverse Events**

| | ET743 0.9 mg/m² (N=3) | ET743 1.1 mg/m² (N=12) | ET743 1.3 mg/m² (N=6) | Total (N=30) |
|---|---|---|---|---|
| **Total no. subjects with Grade 3/4 drug-re!ated AE-** | 0 | 10 | 6 | 17- |
| **Liver and Biliary System** | 0 | 6 | 3 | 9 |
| SGPT Increased | 0 | 6 | 3* | 9 |
| SGOT Increased | 0 | 1 | 2* | 3 |
| **Other** | 0 | 4 | 2 | 7 |
| Palmar-Plantar Erythrodysesthesia | 0 | 2 | 2 | 4 |
| Allergic Reaction | 0 | 2 | 0 | 2 |
| Nausea | 0 | 1 | 0 | 2 |

| | | | | |
|---|---|---|---|---|
| * DLT (dose-limiting toxicity): 2 patients experienced grade 4 elevation of SGPT during cycle 1. | | | | |

In addition, Tables 6a and 6b show the drug-related serious adverse events reported.

**Table 6a Drug Related Serious Adverse Events**

| | | | | |
|---|---|---|---|---|
| | ET743 0.4 mg/m² (N=3) | ET743 0.6 mg/m² (N=3) | | ET743 0.75 mg/m² (N=3) |
| **Total no. subjects with SAE** | 0 | 0 | | 0 |
| Nausea/Vomiting | 0 | 0 | | 0 |
| **Table 6b Drug Related Serious Adverse Events** | | | | |
| | ET743 0.9 mg/m² (N=3) | ET743 1.1 mg/m² (N=12) | ET743 1.3 mg/m² (N=6) | Total (N=30) |
| **Total no. subjects with SAE** | 0 | 1 | 0 | 1 |
| Nausea/Vomiting | 0 | 1 | 0 | 1 |

Out of 18 subjects that discontinued treatment only one subject terminated treatment due to drug related adverse event (Tables 7a and 7b).

**Table 7a: Subject Disposition/Reasons For Treatment Termination**

| | ET743 0.4 mg/m² (N=3) | ET743 0.6 mg/m² (N=3) | ET743 0.75 mg/m² (N=3) |
|---|---|---|---|
| **Ongoing (Cycles)** | **1 (27+)** | **1 (24+)** | **1 (19+)** |
| **Terminated** | 2 | 2 | 2 |
| **Death** | 0 | 0 | 0 |
| **Drug-Related AE** (hand-foot syndrome) | 0 | 0 | 0 |
| **Disease Progression** | 2 | 1 | 2 |
| **Subject Choice** | 0 | 1 | 0 |

**Table 7b : Subject Disposition/Reasons For Treatment Termination**

| | ET743 0.9 mg/m² (N=3) | ET743 1.1 mg/m² (N=12) | ET743 1.3 mg/m² (N=6) | Total (N=30) |
|---|---|---|---|---|
| **Ongoing (Cycles)** | **0** | **6 (8+)** | **3 (13+)** | **12** |
| **Terminated** | 3 | 6 | 3 | 18 |
| **Death** | 0 | 0 | 1 | 1 |
| **Drug-Rotated AE** (hand-foot syndrome) | 0 | 1 | 0 | 1 |
| **Disease Progression** | 3 | 5 | 2 | 15 |
| **Subject Choice** | 0 | 0 | 0 | 1 |

Five patients, three with soft tissue sarcoma , and one each of ovarian and head and neck cancer, had a partial response. Fourteen (14) additional patients (five with sarcoma, and one each carcinoid tumor, pancreatic, bladder, head and neck, thyroid, breast, gastric, SCLC and ovarian cancer) have had stable disease for > 3 months (Table 8a and 8b).

**Table 8a: Best Overall Response**

| **Best Response** | ET743 0.4 mg/m² (N=3) | ET743 0.6 mg/m² (N=3) | ET743 0.75 mg/m² (N=3) |
|---|---|---|---|
| PR | 1 Sarcoma | 0 | 0 |
| | | 1 Sarcoma | |
| SD | 1 Pancreatic 1 Carcinoid tumor | 1 Bladder 1 Head & Neck | 1 Thyroid |

**Table 8b: Best Overall Response**

| **Best Response** | ET743 0.9. mg/m² (N=3) | ET743 1.1 mg/m² (N=12) | ET743 1.3 mg/m² (N=6) |
|---|---|---|---|
| | | | 1 Papillary serous AC |
| PR | 0 | 1 Sarcoma (PNET) | 1 Sarcoma |
| | | | 1 Head & Neck |
| | | 3 Sarcoma | |
| SD | 0 | 1 Breast | 1 Ovarian |
| | | 1 Gastric | 1 Sarcoma |
| | | 1 SCLC | |

The concomitant administration of PLD does not have an impact on the pharmacokinetics (PK) of trabectedin. Based on preliminary pharmacokinetic analysis, the values of trabectedin CL (systematic clearance after i.v. dose), t1/2 (half life) and Vss (apparent volume of distribution at steady state) are within the range observed when trabectedin is given alone (historical control data) (Figure 1, Table 9).

**Table 9: Mean Noncompartmental Pharmacokinetic Parameters of Trabectedin**

| | Dose (mg/m²) | N | Cₘₐₓ (ng/ml) | AUC_{∞} (ng*h/ml) | Cl (1/h/m2) | Vss (1/m²) | T_{1/2} (h) |
|---|---|---|---|---|---|---|---|
| PLD 30 mg/m² | 0.9 | 3 | 6 | 39 | 23 | 1425 | 93 |
| 1 hour infusion + ET-7433 hours | 1.1 | 3 | 6 | 42 | 26 | 1113 | 65 |
| infusion | 1.3 | 2 | 7 | 31 | 44 | 962 | 47 |

From this study we conclude that the MTD of trabectidin is 1.1 mg/m² when is administered in combination with PLD 30 mg/m². It has been demonstrated that this combination is well tolerated when both drugs are administered at full (or near full) therapeutic doses for prolonged periods of time. The recommended dose of this combination treatment is 1.1 mg/m² of trabectidin plus 30 mg/m² of PDL.

In addition, it has been shown that the pharmacokinetics of trabectidin were overtly not impacted by concomitant administration of PDL.

## Claims

1. The use of ET-743 in the preparation of a medicament for an effective treatment for cancer of the human body by combination therapy employing an effective therapeutic amount of ET-743 with an effective therapeutic amount of Pegylated Liposomal form of the anthracycline Doxorubicin ("PLD").

2. The use of PLD in the preparation of a medicament for an effective treatment for cancer of the human body by combination therapy employing an effective therapeutic amount of PLD with an effective therapeutic amount of ET-743.

3. The use according to claim 1 or claim 2, wherein the combination of ET-743 with PLD is synergistic.

4. The use According to any one of the preceding claims, wherein said effective therapeutic amounts of ET-743 and PLD are to be administered as part of the same medicaments or are provided as separate medicaments to be administered at the same time or at different times.

5. The use according to claim 4, wherein said effective therapeutic amounts of ET-743 and PLD are provided as separate medicaments to be administered at different times.

6. The use According to claim 5, wherein said effective therapeutic amount of PLD is to be administered prior to the Administration of said effective therapeutic amount of ET-743.

7. The use according to any one of the preceding claims, wherein said effective therapeutic amounts of PLD and ET-743 are to be administered by intravenous injection.

8. The use according to claim 7, wherein the infusion time for intravenous injection is up to 6 hours for said effective therapeutic amount of PLD and up to 24 hours for said effective therapeutic amount of ET-743.

9. The use according to claim 8, wherein the infusion time for intravenous injection is 1-2 hours for said effective therapeutic amount of PLD and about 3:hours for said effective therapeutic amount of ET-743.

10. The use According to any one of claims 7 to 9, wherein the infusions are to be carried out at an interval of 3 to 4 weeks.

11. The use according to any one of the preceding claims, wherein an effective therapeutic amount of PLD is to be administered in a dosage of up to 50 mg/m², followed by an effective therapeutic amount of ET-743 to be administered in a dosage of up to 1.3 mg/m².

12. The use according to claim 11, wherein said effective therapeutic amount of PLD is to be administered in a dosage of 30-40 mg/m² over an infusion time of 1-2 hours followed by said effective therapeutic amount of ET-743 to be administered in a dosage of about 1.1 mg/m² over an infusion time of about 3 hours.

13. The use according to claim 12, wherein said effective therapeutic amount of PLD is to be administered in a dosage of 30 mg/m² over an infusion time of about 1 hour followed by said effective therapeutic amount of ET-743, to be administered in a dosage of 1.1 mg/m² over an infusion time of about 3 hours.

14. The use according to any one of the preceding claims, wherein the patient has a cancer selected from soft tissue Sarcoma, ovarian cancer and head and neck cancer.

15. A medical kit for administering BT-743 in combination with PLD, comprising a supply of ET-743 in dosage units for at least one cycle, wherein the dosage unit contains the appropriate amount of ET-743 for a use according to any one of claims 1 to 14 and a pharmaceutically acceptable carrier, and printed instructions for administering ET-743 in a use according to any one of claims 1 to 141.

16. A medical kit for administering ET-743 in combination with PLD, comprising a supply of ET-743 in dosage units, wherein each of said dosage units contains an amount of ET-743 for its therapeutically effective administration in combination with PLD.

17. A medical kit for administering PLD in combination with ET-743, comprising a supply of PLD in dosage units, wherein each of said dosage units contains an amount of PLD for its therapeutically effective administration in combinations with ET-743;

18. A medical kit for administering ET-743 in combination with PLD, comprising both a supply of FT-743 according to claim 16 and a supply of PLD according to claim 17.

19. A medical kit according to claim 16 or 18, further comprising instructions for administering ET-743 According to a dosing schedule.

20. A medical kit according to claim 17 or 18, further comprising instructions for administering PLD according to a dosing schedule.

21. A combination for use in the treatment of cancer of the human body, comprising effective therapeutic amounts of ET-743 and PLD which are part of the same medicament or are provided as separate medicaments for the administration at the same time or at different times.

22. A combination according to claim 21, wherein said effective therapeutic amounts of ET-743 and PLD are provided as separate medicaments for the administration at the same time or at different times.

23. A combinations according to claims 22, wherein said effective therapeutic amounts of ET-743 and PLD are provided as separate medicaments for the administration at different times.

24. A combination according to claim 23, wherein said effective amount of PLD is to be administered prior to the administration of said effective therapeutic amount of ET-743.

25. A combination according to any one of claims 21 to 24, wherein said effective therapeutic amounts of PLD and ET-743 are to be administered by intravenous injection.

26. A pharmaceutical composition comprising a therapeutically effective amount of ET743, a therapeutically effective amounts or doxorubicin and a pharmaceutical carrier, wherein doxorubicin is formulated in Pegylated Liposomal form.

## Patentansprüche

1. Verwendung von ET-743 bei der Herstellung eines Medikaments für eine wirksame Behandlung für Krebs des menschlichen Körpers durch Kombinationstherapie, indem eine wirksame therapeutische Menge von ET-743 mit einer wirksamen therapeutischen Menge einer pegylierten liposomalen Form des Anthracyclins Doxorubicin ("PLD") angewendet wird.

2. Verwendung von PLD bei der Herstellung eines Medikaments für eine wirksame Behandlung für Krebs des menschlichen Körpers durch Kombinationstherapie, indem eine wirksame therapeutische Menge von PLD mit einer wirksamen therapeutischen Menge von ET-743 angewendet wird.

3. Verwendung nach Anspruch 1 oder Anspruch 2, wobei die Kombination von ET-743 mit PLD synergistisch ist.

4. Verwendung nach einem der vorhergehenden Ansprüche, wobei die wirksamen therapeutischen Mengen von ET-743 und PLD als Teil des gleichen Medikaments verabreicht werden sollen oder als gesonderte Medikamente bereitgestellt werden, die zur gleichen Zeit oder zu verschiedenen Zeiten verabreicht werden sollen.

5. Verwendung nach Anspruch 4, wobei die wirksamen therapeutischen Mengen von ET-743 und PLD als gesonderte Medikamente bereitgestellt werden, die zu verschiedenen Zeiten verabreicht werden sollen.

6. Verwendung nach Anspruch 5, wobei die wirksame therapeutische Menge von PLD vor der Verabreichung der wirksamen therapeutischen Menge von ET-743 verabreicht werden soll.

7. Verwendung nach einem der vorhergehenden Ansprüche, wobei die wirksamen therapeutischen Mengen von PLD und ET-743 durch intravenöse Injektion verabreicht werden sollen.

8. Verwendung nach Anspruch 7, wobei die Infusionszeit für intravenöse Injektion für die wirksame therapeutische Menge von PLD bis zu 6 Stunden beträgt und für die wirksame therapeutische Menge von ET-743 bis zu 24 Stunden beträgt.

9. Verwendung nach Anspruch 8, wobei die Infusionszeit für intravenöse Injektion für die wirksame therapeutische Menge von PLD 1-2 Stunden und für die wirksame therapeutische Menge von ET-743 etwa 3 Stunden beträgt.

10. Verwendung nach einem der Ansprüche 7 bis 9, wobei die Infusionen in einem Intervall von 3 bis 4 Wochen ausgeführt werden sollen.

11. Verwendung nach einem der vorhergehenden Ansprüche, wobei eine wirksame therapeutische Menge von PLD in einer Dosierung von bis zu 50 mg/m² verabreicht werden soll, gefolgt von einer wirksamen therapeutischen Menge von ET-743, die in einer Dosierung von bis zu 1,3 mg/m² verabreicht werden soll.

12. Verwendung nach Anspruch 11, wobei die wirksame therapeutische Menge von PLD in einer Dosierung von 30-40 mg/m² über eine Infusionszeit von 1-2 Stunden verabreicht werden soll, gefolgt von der wirksamen therapeutischen Menge von ET-743, die in einer Dosierung von etwa 1,1 mg/m² über eine Infusionszeit von etwa 3 Stunden verabreicht werden soll.

13. Verwendung nach Anspruch 12, wobei die wirksame therapeutische Menge von PLD in einer Dosierung von 30 mg/m² über eine Infusionszeit von etwa 1 Stunde verabreicht werden soll, gefolgt von der wirksamen therapeutischen Menge von ET-743, die in einer Dosierung von 1,1 mg/m² über eine Infusionszeit von etwa 3 Stunden verabreicht werden soll.

14. Verwendung nach einem der vorhergehenden Ansprüche, wobei der Patient einen Krebs, ausgewählt aus Weichteilsarkom, Eierstockkrebs und Kopf- und Halskrebs, hat.

15. Medizinischer Kit zum Verabreichen von ET-743 in Kombination mit PLD, umfassend eine Zuführung von ET-743 in Dosierungseinheiten für mindestens einen Zyklus, wobei die Dosierungseinheit die passende Menge von ET-743 für eine Verwendung nach einem der Ansprüche 1 bis 14 und einen pharmazeutisch verträglichen Träger sowie gedruckte Instruktionen zum Verabreichen von ET-743 in einer Verwendung nach einem der Ansprüche 1 bis 14 enthält.

16. Medizinischer Kit zum Verabreichen von ET-743 in Kombination mit PLD, umfassend eine Zuführung von ET-743 in Dosierungseinheiten, wobei jede der Dosierungseinheiten eine Menge von ET-743 für ihre therapeutisch wirksame Verabreichung in Kombination mit PLD enthält.

17. Medizinischer Kit zum Verabreichen von PLD in Kombination mit ET-743, umfassend eine Zuführung von PLD in Dosierungseinheiten, wobei jede der Dosierungseinheiten eine Menge von PLD für ihre therapeutisch wirksame Verabreichung in Kombination mit ET-743 enthält.

18. Medizinischer Kit zum Verabreichen von ET-743 in Kombination mit PLD, umfassend sowohl eine Zuführung von ET-743 nach Anspruch 16 als auch eine Zuführung von PLD nach Anspruch 17.

19. Medizinischer Kit nach Anspruch 16 oder 18, weiterhin umfassend Instruktionen zum Verabreichen von ET-743 nach einem Dosierungsschema.

20. Medizinischer Kit nach Anspruch 17 oder 18, weiterhin umfassend Instruktionen zum Verabreichen von PLD nach einem Dosierungsschema.

21. Kombination zur Verwendung bei der Behandlung von Krebs des menschlichen Körpers, umfassend wirksame therapeutische Mengen von ET-743 und PLD, welche Teil des gleichen Medikaments sind oder als gesonderte Medikamente bereitgestellt werden, für die Verabreichung zur gleichen Zeit oder zu verschiedenen Zeiten.

22. Kombination nach Anspruch 21, wobei die wirksamen therapeutischen Mengen von ET-743 und PLD als gesonderte Medikamente für die Verabreichung zur gleichen Zeit oder zu verschiedenen Zeiten bereitgestellt werden.

23. Kombination nach Anspruch 22, wobei die wirksamen therapeutischen Mengen von ET-743 und PLD als gesonderte Medikamente für die Verabreichung zu verschiedenen Zeiten bereitgestellt werden.

24. Kombination nach Anspruch 23, wobei die wirksame Menge von PLD vor der Verabreichung der wirksamen therapeutischen Menge von ET-743 verabreicht werden soll.

25. Kombination nach einem der Ansprüche 21 bis 24, wobei die wirksamen therapeutischen Mengen von PLD und ET-743 durch intravenöse Injektion verabreicht werden sollen.

26. Pharmazeutische Zusammensetzung, umfassend eine therapeutisch wirksame Menge von ET-743, eine therapeutisch wirksame Menge von Doxorubicin und einen pharmazeutischen Träger, wobei Doxorubicin in pegylierter liposomaler Form formuliert wird.

## Revendications

1. Utilisation de ET-743 dans la préparation d'un médicament pour un traitement efficace destiné à un cancer du corps humain par une thérapie de combinaison employant une quantité thérapeutique efficace de ET-743 avec une quantité thérapeutique efficace d'une forme liposomiale pégylée de l'anthracycline doxorubicine (« PLD »).

2. Utilisation de PLD dans la préparation d'un médicament pour un traitement efficace destiné à un cancer du corps humain par une thérapie de combinaison employant une quantité thérapeutique efficace de PLD avec une quantité thérapeutique efficace de ET-743.

3. Utilisation selon la revendication 1 ou la revendication 2, dans laquelle la combinaison de ET-743 avec PLD est synergique.

4. Utilisation selon l'une quelconque des revendications précédentes, dans laquelle lesdites quantités thérapeutiques efficaces de ET-743 et de PLD sont à administrer comme une partie du même médicament ou sont fournies comme des médicaments séparés à administrer au même moment ou à des moments différents.

5. Utilisation selon la revendication 4, dans laquelle lesdites quantités thérapeutiques efficaces de ET-743 et de PLD sont fournies comme des médicaments séparés à administrer à des moments différents.

6. Utilisation selon la revendication 5, dans laquelle ladite quantité thérapeutique efficace de PLD est à administrer avant l'administration de ladite quantité thérapeutique efficace de ET-743.

7. Utilisation selon l'une quelconque des revendications précédentes, dans laquelle lesdites quantités thérapeutiques efficaces de PLD et de ET-743 sont à administrer par une injection intraveineuse.

8. Utilisation selon la revendication 7, dans laquelle le temps de perfusion pour l'injection intraveineuse est jusqu'à 6 heures pour ladite quantité thérapeutique efficace de PLD et jusqu'à 24 heures pour ladite quantité thérapeutique efficace de ET-743.

9. Utilisation selon la revendication 8, dans laquelle le temps de perfusion pour l'injection intraveineuse est de 1-2 heures pour ladite quantité thérapeutique efficace de PLD et d'environ 3 heures pour ladite quantité thérapeutique efficace de ET-743.

10. Utilisation selon l'une quelconque des revendications 7 à 9, dans laquelle les perfusions sont à réaliser à un intervalle de 3 à 4 semaines.

11. Utilisation selon l'une quelconque des revendications précédentes, dans laquelle une quantité thérapeutique efficace de PLD est à administrer en un dosage de jusqu'à 50 mg/m², suivie par une quantité thérapeutique efficace de ET-743, à administrer en un dosage de jusqu'à 1,3 mg/m².

12. Utilisation selon la revendication 11, dans laquelle ladite quantité thérapeutique efficace de PLD est à administrer en un dosage de 30-40 mg/m² sur un temps de perfusion de 1-2 heures, suivie par ladite quantité thérapeutique efficace de ET-743, à administrer en un dosage d'environ 1,1 mg/m² sur un temps de perfusion d'environ 3 heures.

13. Utilisation selon la revendication 12, dans laquelle ladite quantité thérapeutique efficace de PLD est à administrer en un dosage de 30 mg/m² sur un temps de perfusion d'environ 1 heure, suivie par ladite quantité thérapeutique efficace de ET-743, à administrer en un dosage de 1,1 mg/m² sur un temps de perfusion d'environ 3 heures.

14. Utilisation selon l'une quelconque des revendications précédentes, dans laquelle le patient présente un cancer choisi parmi un sarcome des tissus mous, un cancer de l'ovaire et un cancer de la tête et du cou.

15. Kit médical pour l'administration de ET-743 en combinaison avec PLD, comprenant un approvisionnement de ET-743 dans des unités de dosage pour au moins un cycle, dans lequel l'unité de dosage contient la quantité appropriée de ET-743 pour une utilisation selon l'une quelconque des revendications 1 à 14, et un véhicule pharmaceutiquement acceptable et des instructions imprimées pour l'administration de ET-743 dans une utilisation selon l'une quelconque des revendications 1 à 14.

16. Kit médical pour l'administration de ET-743 en combinaison avec PLD, comprenant un approvisionnement de ET-743 dans des unités de dosage, dans lequel chacune desdites unités de dosage contient une quantité de ET-743 pour son administration thérapeutiquement efficace en combinaison avec PLD.

17. Kit médical pour l'administration de PLD en combinaison avec ET-743, comprenant un approvisionnement de PLD dans des unités de dosage, dans lequel chacune desdites unités de dosage contient une quantité de PLD pour son administration thérapeutiquement efficace en combinaison avec ET-743.

18. Kit médical pour l'administration de ET-743 en combinaison avec PLD, comprenant à la fois un approvisionnement de ET-743 selon la revendication 16 et un approvisionnement de PLD selon la revendication 17.

19. Kit médical selon la revendication 16 ou 18, comprenant en outre des instructions pour l'administration de ET-743 selon un programme de dosage.

20. Kit médical selon la revendication 17 ou 18, comprenant en outre des instructions pour l'administration de PLD selon un programme de dosage.

21. Combinaison pour une utilisation dans le traitement d'un cancer du corps humain, comprenant des quantités thérapeutiques efficaces de ET-743 et de PLD qui sont des parties du même médicament ou sont fournies comme des médicaments séparés pour une administration au même moment ou à des moments différents.

22. Combinaison selon la revendication 21, dans laquelle lesdites quantités thérapeutiques efficaces de ET-743 et de PLD sont fournies comme des médicaments séparés pour une administration au même moment ou à des moments différents.

23. Combinaison selon la revendication 22, dans laquelle lesdites quantités thérapeutiques efficaces de ET-743 et de PLD sont fournies comme des médicaments séparés pour une administration à des moments différents.

24. Combinaison selon la revendication 23, dans laquelle ladite quantité efficace de PLD est à administrer avant l'administration de ladite quantité thérapeutique efficace de ET-743.

25. Combinaison selon l'une quelconque des revendications 21 à 24, dans laquelle lesdites quantités thérapeutiques efficaces de PLD et de ET-743 sont à administrer par une injection intraveineuse.

26. Composition pharmaceutique comprenant une quantité thérapeutiquement efficace de ET-743, une quantité thérapeutiquement efficace de doxorubicine et un véhicule pharmaceutique, dans laquelle la doxorubicine est formulée sous une forme liposomiale pégylée.
